# EUROPÄISCHE PATENTANMELDUNG

(11) **EP 1 306 099 A2**
(43) Veröffentlichungstag der Anmeldung: **02.05.2003**
(21) Anmeldenummer: 03001806.3
(22) Anmeldetag: 02.09.1995
(51) Int. Cl.: A61M 16/00, A61B 5/085

(54) **Verfahren zur Steuerung eines Beatmungsgerätes sowie Beatmungsgerät**

(30) Priorität: 08.09.1994 DE 9414568 U
(62) Teilanmeldung aus: 95113816.3
(71) Anmelder: GOTTLIEB WEINMANN GERÄTE FÜR MEDIZIN UND ARBEITSSCHUTZ GMBH & CO., 22525 Hamburg (DE)
(72) Erfinder: Graetz, Bernd, 22869 Schenefeld (DE); Maurer, Jörg, 21521 Aumühle (DE)
(74) Vertreter: Klickow, Hans-Henning

(57) **Zusammenfassung**

Das Verfahren dient zur Steuerung eines Beatmungsgerätes. Das Beatmungsgerät wird zur Atmungsunterstützung bei einem Auftreten von Symptomen der Schlafapnoe eingesetzt. Unter Verwendung der Oszilloresistometrie, die auch als oszillatorische Resistenz-Messung bezeichnet wird, wird die oszillatorische Druckamplitude in der Luftzuführung eines Patienten kontinuierlich gemessen. Die oszillatorische Druckamplitude korrespondiert zu einem Atemwiderstand des Patienten. Nach einem Bestimmen einer oszillatorischen Druckamplitude als Basiswert, die einem individuellen Atemwiderstandswert entspricht, wird beim Überschreiten von Grenzwerten für Abweichungen von diesem Basiswert dem Patienten Atemgas unter einem erhöhten Druck zugeführt. Der Druck wird wieder reduziert oder minimiert, sobald der Basiswert der oszillatorischen Druckamplitude wieder oder annähernd erreicht ist.

Das Beatmungsgerät dient zu einer Gerätetechnischen Realisierung des Verfahrens.

## Beschreibung

Die Erfindung betrifft ein Verfahren zur Steuerung eines Beatmungsgerätes, das zur Atmungsunterstützung bei einem Auftreten von Symptomen der Schlafapnoe eingesetzt wird.

Die Erfindung betrifft darüber hinaus ein Beatmungsgerät zur Atmungsunterstützung bei einem Auftreten von Symptomen der Schlafapnoe, bestehend aus einer mit einer Atemmaske verbundenen Druckgasquelle.

Eine nicht unbeachtliche Anzahl von Menschen leiden unter Schlafstörungen, die sich auf das Tagesbefinden dieser Menschen auswirken und deren soziale und berufliche Leistungsfähigkeit sowie deren Lebensqualität zum Teil erheblich beeinträchtigen. Eine dieser Schlafstörungen ist die Schlafapnoe, die vorrangig mit der sogenannten CPAP-Therapie (CPAP = Continuous Positive Airway Pressure) behandelt wird, indem dem Patienten während des Schlafes ein Luftstrom aus einem atemfähigen Gas über eine Nasalmaske kontinuierlich zugeführt wird. Die Maske ist über einen Schlauch mit einem Beatmungsgerät verbunden, das einen Lüfter umfaßt, der einen Gasstrom mit einem Überdruck von 5 bis 20 mbar erzeugt.

Der Gasstrom wird dem Patienten entweder mit konstantem Druck zugeführt, oder zur Erleichterung der Atemarbeit des Patienten beim Ausatmen auf ein niedrigeres Druckniveau abgesenkt. Obwohl Schlafapnoen nur kurzfristig auftreten und einen geringen Teil des Schlafes ausmachen, läuft der Lüfter bei beiden Verfahren während der gesamten Schlafdauer (Nacht), was die Akzeptanz dieser Schlafapnoe-Behandlung erschwert.

Aus der US-A-5 245 995 ist ein CPAP-Beatmungsgerät bekannt, das für Patienten mit Schlafapnoe einsetzbar ist. Das Atemgas wird dem Patienten über eine Atemmaske zugeführt und im Bereich des Gerätes ist eine Druckgasquelle angeordnet. Die Druckgasquelle ist in Abhängigkeit vom Atemwiderstand steuerbar.

Aus der EP-A-0 373 585 ist es bekannt, den Atemwiderstand eines Patienten mit Hilfe der ORM-Messung durchzuführen. Mit vorgebbarer Frequenz wird hierbei dem Atemvolumenstrom ein oszillierender Volumenstrom mit geringem Volumenhub überlagert. Aus der mit gleicher Frequenz auftretenden periodischen Druckschwankung kann ein vom konkreten Atemwiderstand abhängiger Meßwert bereitgestellt werden.

Aus der US-A-5 318 038 ist es bekannt-, eine Atemmessung unter Verwendung eines Pneumotachographen in der Gaszuführleitung durchzuführen.

Aufgabe der Erfindung ist es nun, die CPAP-Therapie patientenfreundlich zu gestalten.

Diese Aufgabe wird erfindungsgemäß dadurch gelöst, daß mit der Oszilloresistometrie (oszillatorische Resistenz-Messung = ORM) die oszillatorische Druckamplitude in der Luftzuführung eines Patienten, die zu einem Atemwiderstand des Patienten korrespondiert, kontinuierlich gemessen wird, wobei nach dem Bestimmen einer einem individuellen Atemwiderstandswert als Basiswert entsprechenden oszillatorischen Druckamplitude bei Überschreiten von Grenzwerten für Abweichungen von diesem Basiswert dem Patienten Atemgas unter einem erhöhten Druck zugeführt und der Druck wieder reduziert oder minimiert wird, sobald der Basiswert der oszillatorischen Druckamplitude wieder oder annähernd erreicht ist.

Weitere Aufgabe der vorliegenden Erfindung ist es, ein Beatmungsgerät der einleitend genannten Art derart zu konstruieren, daß eine verbesserte Benutzungsfreundlichkeit bereitgestellt ist.

Diese Aufgabe wir erfindungsgemäß dadurch gelöst, daß eine Einrichtung zur kontinuierlichen Messung der einem Atemwiderstand eines Patienten entsprechenden oszillatorischen Druckamplitude nach dem ORM-Prinzip vorgesehen ist, die mit einer Steuer-Regeleinrichtung verbunden ist, die auf einen Basiswert der oszillatorischen Druckamplitude einstellbar ist, der einem individuellen Atemwiderstandsbeiwert des Patienten entspricht und bei der die Steuer-Regeleinrichtung die Druckgasquelle derart aktiviert bzw. steuert, daß dem Patienten bei vorgebbaren Abweichungen der oszillatorischen Druckamplitude vom Basiswert Atemgas mit erhöhtem Druck zugeführt wird.

Bei dem erfindungsgemäßen Verfahren zur Steuerung und Regelung eines Beatmungsgerätes zur Atmungsunterstützung von Schlafapnoe-Patienten wird dieses Gerät nur dann aktiviert, d.h. es wird dem Patienten nur dann Atemgas zugeführt, wenn durch eine Apnoe die Atemtätigkeit des Patienten gestört ist oder sich der Atemwiderstand - und somit auch die gemessene oszillatorische Druckamplitude - durch eine entstehende Apnoe ändert. Eine Störung der Atemtätigkeit des Patienten ist von einer Veränderung des Atemwiderstandes des Patienten begleitet. Dieser Widerstand wird ohne das Befinden des Patienten zu beeinflussen, auf einfache Weise zuverlässig und reproduzierbar über die Veränderungen der oszillatorischen Druckamplitude bestimmt. Diese bildet die Steuergröße für das Ein- und Ausschalten des Behandlungsgerätes, so daß eine Unterversorgung des Patienten mit Sauerstoff nicht eintritt.

Das Befinden des Patienten kann bei einer vorteilhaften Weiterbildung des erfindungsgemäßen Verfahrens noch dadurch gesteigert werden, daß dem Patienten ständig Atemgas mit einem mäßigen Grunddruck von 3 bis 5 mbar zugeführt, der Gasdruck bei Abweichungen vom individuellen Atemwiderstandswert als Basiswert angehoben und wieder auf den Grunddruck abgesenkt wird, sobald der Basiswert wieder annähernd erreicht ist. Diese kontinuierliche Gaszufuhr unterstützt nicht nur die Atemarbeit des Patienten wohltuend, sondern wirkt sich sogleich vorteilhaft auf die Ermittlung der zur Steuerung des Druckes des Atemgases erforderlichen Parameter aus.

Im Verlauf einer beginnenden Apnoe bewirken Verengungen oder Erweiterungen der Atemwege eines Patienten Änderungen im Phasenwinkel der oszillatorischen Druckamplitude und des Atemflusses im Vergleich zu den entsprechenden Basiswerten bei normalem oder ungestörtem Atemhub. Diesen unerwünschten Veränderungen kann mit weiteren zweckmäßigen Ausgestaltungen der Erfindung dadurch begegnet werden, daß als zusätzliche Steuer- und/oder Regelsignale für das Beatmungsgerät der Phasenwinkel der Druckamplitude und/oder der Atemfluß des Patienten herangezogen werden, um bei signifikanten Abweichungen von den jeweiligen - zu Beginn der Therapie ermittelten - individuellen Basiswerten den Druck des zugeführten Atemgases so zu erhöhen, bis die Basiswerte wieder oder annähernd erreicht sind.

Ein Beatmungsgerät zur Durchführung des Verfahrens nach der Erfindung besteht aus einer mit einer Atemmaske verbundenen und zweckmäßigerweise als Lüfter ausgebildeten Druckgasquelle und ist durch eine Einrichtung zur kontinuierlichen Messung der oszillatorischen Druckamplitude eines Patienten nach dem ORM-Prinzip sowie durch eine Steuer-Regeleinrichtung gekennzeichnet, die auf den individuellen Basiswert der dem Atemwiderstandswert eines Patienten entsprechenden oszillatorischen Druckamplitude einstellbar ist und die Druckgasquelle so aktiviert, daß dem Patienten Atemgas zugeführt wird, wenn signifikante Abweichungen des Atemwiderstandes vom Basiswert auftreten.

Bei einer zweckmäßigen Ausgestaltung kann das Beatmungsgerät als weitere Steuerelemente zum Aktivieren der Druckgasquelle noch eine Einrichtung zum Messen und Bestimmen des Phasenwinkels der Druckamplitude und/oder eine als Pneumotachograph oder Meßblende ausgebildete Einrichtung zum Bestimmen des Atemflusses des Patienten aufweisen.

Bei einer anderen Weiterbildung der Erfindung kann das Beatmungsgerät noch mit einem auf die Bedürfnisse des Patienten einstellbaren Druckregler für das Atemgas versehen sein, wodurch die Akzeptanz des Therapiegerätes gesteigert wird, da der Patient mit einem für ihn angenehmen konstanten Druck beatmet wird, wenn die oszillatorische Druckamplitude und/oder der Phasenwinkel der Druckamplitude und/oder der Atemfluß des Patienten dem (den) Basiswert(en) entspricht (entsprechen).

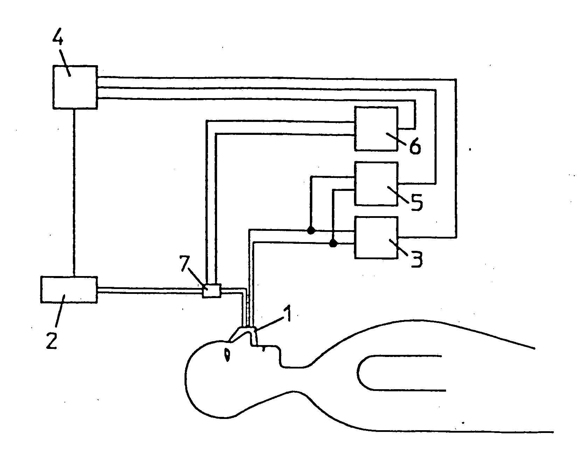

Ein Ausführungsbeispiel des erfindungsgemäßen Beatmungsgerätes wird noch an Hand der Zeichnung, in der es schematisch dargestellt ist, beschrieben.

Das Beatmungsgerät zur Therapie der Schlafapnoe weist eine auf der Nase eines Patienten anordbare Atemmaske (1) auf, die über einen Atemschlauch (8) mit einer als Lüfter ausgebildeten Druckgasquelle (2) verbunden ist. In der Atemmaske (1) sind die Sensoren (nicht gezeigt) einer Einrichtung (3) zum kontinuierlichen Messen der oszillatorischen Druckamplitude nach dem ORM-Prinzip vorgesehen. Von einer Steuer-Regeleinrichtung (4), die mit der Einrichtung (3) verbunden und auf den individuellen Basiswert der dem Atemwiderstand des Patienten entsprechenden oszillatorischen Druckamplitude einstellbar ist, wird die Druckgasquelle (2) so aktiviert, daß dem Patienten Atemgas bei signifikanten Abweichungen der Druckamplitude vom Basiswert zugeführt wird. In den Atemschlauch (8) ist eine Meßblende (7) eingesetzt, die den Atemfluß erfaßt und die entsprechenden Daten über eine Meßeinrichtung (6) an die Steuer-Regeleinrichtung (4) zur weiteren Beeinflussung der Druckgasquelle (2) weiterleitet. In der Atemmaske (1) sind außerdem noch die Sensoren (nicht dargestellt) einer anderen Einrichtung (5) zum Messen des Phasenwinkeis der Druckamplitude vorgesehen. Die Einrichtung (5) sendet an die Steuer-Regeleinrichtung (4) Signale, die als zusätzliche Regelparameter bei der Aktivierung der Druckquelle (2) dienen.

## Patentansprüche

1. Verfahren zur Steuerung eines Beatmungsgerätes, das zur Atmungsunterstützung bei einem Auftreten von Symptomen der Schlafapnoe eingesetzt wird, **dadurch gekennzeichnet, daß** mit der Oszilloresistometrie (oszillatorische Resistenz-Messung = ORM) die oszillatorische Druckamplitude in der Luftzuführung eines Patienten, die zu einem Atemwiderstand des Patienten korrespondiert, kontinuierlich gemessen wird, wobei nach dem Bestimmen einer einem individuellen Atemwiderstandswert als Basiswert entsprechenden oszillatorischen Druckamplitude bei Überschreiten von Grenzwerten für Abweichungen von diesem Basiswert dem Patienten Atemgas unter einem erhöhten Druck zugeführt und der Druck wieder reduziert oder minimiert wird, sobald der Basiswert der oszillatorischen Druckamplitude wieder oder annähernd erreicht ist.

2. Verfahren nach Anspruch 1, **dadurch gekennzeichnet, daß** dem Patienten ständig Atemgas mit einem Grunddruck von etwa 3 bis 5 mbar zugeführt, der Gasdruck bei Abweichungen von der individuellen oszillatorischen Druckamplitude als Basiswert angehoben und auf den Grunddruck abgesenkt wird, sobald der Basiswert wieder oder annähernd erreicht ist.

3. Verfahren nach Anspruch 1 oder 2, **dadurch gekennzeichnet, daß** der Phasenwinkel zwischen dem oszillatorischen Volumenstrom und dem oszillatorischen Druck in der Luftversorgung des Patienten kontinuierlich gemessen und bei vorgebbaren Abweichungen vom - zu Beginn der Atmungsunterstützung ermittelten - individuellen Basiswert der Druck des Atemgases so erhöht wird, bis der Basiswert wieder oder annähernd erreicht ist.

4. Verfahren nach einem der vorhergehenden Ansprüche, **dadurch gekennzeichnet, daß** der Atemfluß des Patienten gemessen und bei signifikanten Abweichungen vom - zu Beginn der Atmungsunterstützung ermittelten - individuellen Basiswert der Druck des Atemgases so erhöht wird, bis der Basiswert wieder oder annähernd erreicht ist.

5. Beatmungsgerät zur Atmungsunterstützung bei einem Auftreten von Symptomen der Schlafapnoe, bestehend aus einer mit einer Atemmaske verbundenen Druckgasquelle, **dadurch gekennzeichnet, daß** eine Einrichtung (3) zur kontinuierlichen Messung der einem Atemwiderstand eines Patienten entsprechenden oszillatorischen Druckamplitude nach dem ORM-Prinzip vorgesehen ist, die mit einer Steuer-Regeleinrichtung (4) verbunden ist, die auf einen Basiswert der oszillatorischen Druckplitude einstellbar ist, der einem individuellen Atemwiderstandsbeiwert des Patienten entspricht, und bei der die Steuer-Regeleinrichtung (4) die Druckgasquelle (2) derart aktiviert bzw. steuert, daß dem Patienten bei vorgebbaren Abweichungen der oszillatorischen Druckamplitude vom Basiswert Atemgas mit erhöhtem Druck zugeführt wird.

6. Beatmungsgerät nach Anspruch 5, **dadurch gekennzeichnet, daß** die Druckgasquelle (2) ein Lüfter ist.

7. Beatmungsgerät nach Anspruch 5 oder 6, **gekennzeichnet durch** einen auf die Bedürfnisse des Patienten einstellbaren Druckregler für das Atemgas.

8. Beatmungsgerät nach einem der vorhergehenden Ansprüche 5 bis 7, **gekennzeichnet durch** eine Einrichtung (5) zur kontinuierlichen Messung und Bestimmung des Phasenwinkels zwischen dem oszillatorischen Volumenstrom und dem oszillatorischen Druck, welche Einrichtung (5) mit der Steuer-Regeleinrichtung (4) verbunden ist, die auf den individuellen Basiswert des Phasenwinkels des Patienten einstellbare Elemente aufweist.

9. Beatmungsgerät nach einem der Ansprüche 5 bis 8, **gekennzeichnet durch** eine mit einer Meßblende (7) oder einem Pneumotachographen in der Gaszuführleitung (8) verbundene Meßeinrichtung (6) zur kontinuierlichen Messung und Bestimmung des Atemflusses des Patienten, welche Meßeinrichtung (6) mit der Steuer-Regeleinrichtung (4) verbunden ist, die auf den individuellen Basiswert des Atemflusses des Patienten einstellbare Elemente aufweist.

10. Beatmungsgerät nach Anspruch 8 und 9, **dadurch gekennzeichnet, daß** in der Einrichtung (5) zur kontinuierlichen Messung und Bestimmung des Phasenwinkels aus Signalen von der Einrichtung (3) zur kontinuierlichen Messung der oszillatorischen Druckamplitude und Signalen der Meßblende (7) oder des Pneumotachographen der Phasenwinkel zwischen dem oszillatorischen Volumenstrom und dem oszillatorischen Druck bestimmt wird.
